# EUROPÄISCHE PATENTSCHRIFT

(11) **EP 1 216 688 B1**
(45) Veröffentlichungstag und Bekanntmachung des Hinweises auf die Patenterteilung: **24.05.2006**
(21) Anmeldenummer: 01129800.7
(22) Anmeldetag: 14.12.2001
(51) Int. Cl.: A61K 8/21, A61K 8/25, A61K 8/26, A61K 8/34, A61K 8/46, A61K 8/55, A61K 8/73, A61K 8/92, B65D 35/00

(54) **Halbflüssige Zahncreme**
Semifluid dental cream
Crème dentifrice à demi-fluide

(30) Priorität: 23.12.2000 DE 10064986
(43) Veröffentlichungstag der Anmeldung: 26.06.2002
(73) Patentinhaber: Henkel Kommanditgesellschaft auf Aktien, 40589 Düsseldorf (DE)
(72) Erfinder: Leinen, Hans-Theo, Dr., 40229 Düsseldorf (DE); Wülknitz, Peter, Dr., 42799 Leichlingen (DE); Duschek, Nicole, 40595 Düsseldorf (DE); Glasl, Johann, Dr., 42719 Solingen (DE)

(56) Entgegenhaltungen:
- EP-A- 0 255 210
- EP-A- 0 545 678
- EP-A- 0 947 440
- WO-A-01/62209
- DE-A- 1 667 875
- US-A- 4 425 322

## Beschreibung

Gegenstand der Erfindung ist eine Zahnreinigungscreme in Form einer wäßrigen, fließfähigen Dispersion, die sich aufgrund ihrer Zusammensetzung und Viskosität besonders gut zur Ausbringung aus einer flexiblen Spendetube eignet, deren Spendeöffnung ein Schlitzventil aufweist und die in "kopfüber"-Position gelagert werden kann.

Für die Dosierung von pastösen, gel- oder cremeförmigen Zahnreinigungsmitteln sind Tuben aus verformbarem, weichem Metallblech oder aus flexiblen Kunststoffen im Gebrauch. Die für solche Behälter geeigneten Zahnreinigungsmittel sind üblicherweise Pasten, die erst unter dem Einfluß eines auf die Tube ausgeübten Pressdrucks zu fließen beginnen und aus der Tubenöffnung austreten. Daneben sind auch Spendefläschchen aus flexiblen Kunststoffen im Gebrauch, die sich zur Dosierung sogenannter "flüssiger" Zahnreinigungsmittel eignen. Diese Produkte weisen niedrigere Viskositäten und vor allem eine niedrigere Fließgrenze auf, so daß sie bereits unter dem Einfluß der durch das eigene Gewicht erzeugten Schubspannung fließen.

In den letzten Jahren sind darüber hinaus flexible Spendebehälter für pastöse Produkte entwickelt worden, deren Spendeöffnung ein Schlitzventil aufweist. Solche Ventile sind im Ruhezustand geschlossen, unter dem Einfluß eines auf die Tube ausgeübten Preßdrucks geben sie durch elastische Verformung der Verschlußlippen die Spendeöffnung frei und lassen das Produkt austreten. Sobald der Preßdruck nachläßt, schließen sich die elastischen biegsamen Verschlußlippen wieder und verhindern einen weiteren Produktaustritt ebenso wie ein Eindringen von Luft oder Verunreinigung in die Tube. Beispiele solcher Schlitzventile sind in EP 545678 A1, WO 00/24640 A1, EP 0947440 A1, EP 0463658 B1 und EP 0119237 B1 beschrieben. Solche Behälter eignen sich auch zur Dosierung fließfähiger Produkte und zur Lagerung der Behälter in "kopfüber"-Position, d. h. mit der Spendeöffnung nach unten, z. B. wenn die Spendeöffnung mit einer Abdeckung versehen ist, die als Standauflage dient. Auf diese Weise wird eine auf der Abdeckung der Spendeöffnung stehende Lagerung des Behälters ermöglicht. Solche Spendebehälter eignen sich auch für hochviskose Pasten, wenn diese eine ausreichend niedrige Fließgrenze aufweisen und bei der "kopfüber"-Lagerung spontan unter dem Einfluß der Schwerkraft in den Raum vor der Spendeöffnung einfließen und auf diese Weise leicht ausgedrückt und dosiert werden können.

Die üblichen, für konventionelle Tuben bekannten Zahnpasten sind für solche Behälter zu hochviskos bzw. weisen zu hohe Fließgrenzen auf und bereiten Probleme bei der Entleerung, da sie nicht spontan in den Raum vor der Spendeöffnung einfließen. Auch lassen sie sich aufgrund ihrer hohen Viskosität nur schwer durch ein Schlitzventil drücken.

Zwar können die für flexible Spendefläschchen bekannten flüssigen Zahnreinigungsmittel prinzipiell auch aus den Standtuben mit Schlitzventil-Spendeöffnung dosiert werden, es kommt aber zu Problemen des Auslaufens des zu dünnflüssigen Produkts aus dem kopfüber gelagerten Behälter. Auch weist das auf die Zahnbürste dosierte Produkt nicht den Charakter eines Pastenstranges auf und erfüllt daher nicht die ästhetischen Erwartungen des Verwenders einer Zahnpaste.

Es bestand daher die Aufgabe, eine Zahnreinigungscreme zu entwickeln, die sich für die Ausbringung aus einer flexiblen Spendetube mit Schlitzventil-Spendeöffnung und Überkopflagerung eignet.

Aus DE 16 67 875 B2 sind Zahnpasten bekannt, die pyrogene, hydrophobe Kieselsäure und Paraffinöl oder ein pflanzliches Öl anstelle des Feuchthaltemittels enthalten. Aus DE 34 07 860 C2 waren zweiphasige tensidfreie Zahnpflegemittel bekannt, deren hydrophobe Phase Kohlenwasserstofföle oder Pflanzenöle enthält. Aus DE 37 01 123 A1 waren Zahncremes auf Basis von Aluminiumoxid-trihydrat als Poliermittel bekannt, die pflanzliche Öle zur Verhinderung des Angriffs auf Polyolefinoberflächen enthalten. Aus US 5,130,122 sind Zahnpflegemittel bekannt, die eine Mikroemulsion eines pflanzlichen oder mineralischen Öls zur Bindung von Mundgeruch enthalten. Aus WO 91/13608 A1 sind plaquehemmende Zahnpflegemittel bekannt, die ein flüssiges Silikonöl und einen darin gelösten fettlöslichen antibakteriellen Wirkstoff enthalten. Aus WO 01/62209 A2 sind treibgashaltige Zahnreinigungsmittel bekannt, die Poliermittel, Feuchthaltemittel (Glycerin, Sorbit), Tenside und wenigstens eine Öl/Fett- und/oder Wachskomponente (Jojobaöl) enthalten.
Aus US 4,425,322 sind "dual-action"-Zahncreme mit wässriger Phase und Ölphase bekannt, die u.a. Sorbit und Mineralöl enthalten.
Aus EP 0255210 A2 sind Zahncremezusammensetzungen bekannt, die Sorbit, Glycerin, Propylenglycol und Jojoba Alkohol enthalten. Die genannten Produkte sind aber nicht zur Lösung der hier gestellten Aufgabe geeignet.

Es wurde gefunden, daß die gestellte Aufgabe durch eine Erhöhung der Duktilität und Glätte der Zusammensetzung durch einen Gehalt einer dispergierten Ölkomponente und durch Einstellung eines definierten Viskositätsbereichs gelöst werden kann.

Gegenstand der Erfindung ist eine Zahnreinigungscreme in Form einer wäßrigen, fließfähigen Dispersion mit einem Gehalt an Polierkomponenten, Feuchthaltemitteln, Bindemitteln, oberflächenaktiveren Stoffen, Wirkstoffen, Aromen und Hilfsmitteln, dadurch gekennzeichnet, daß 0,1 - 2 Gew.-% wenigstens einer bei 20 °C flüssigen, nicht-essentiellen und nicht-etherischen Ölkomponente darin dispergiert ist und daß die Viskosität bei 20 °C im Bereich von 50-200 Pa·s liegt, wobei als Feuchthaltemittel glycerin, Sorbit und 1,2-Propylenglycol in einem Gewichtsverhältnis von 1:(0,5-1) : (0,1-0,5) und in einer Gesamtmenge von 20-40 gew.-% enthalten Sind.

Als nicht-essentielle und nicht-etherische Ölkomponente kann jede im wesentlichen wasserunlösliche Ölkomponente eingesetzt werden, die organoleptisch in einer Zahnpasta akzeptabel ist und selbst kein Geschmacks- oder Riechstoff ist. Bevorzugt sind die erfindungsgemäß geeigneten Ölkomponenten weitgehend geschmacklos und geruchlos, zumindest sollte ein eventueller Beigeschmack sich durch die in Zahnpasten üblichen Aromen und Geschmackskorrigentien (Süßstoffe) leicht überdecken lassen.

Geeignete Ölkomponenten für die Zwecke der vorliegenden Erfindung sind z. B. Kohlenwasserstoffe (Paraffinöle), Silikonöle, flüssige, ungesättigte oder verzweigte Alkohole mit 12-36 C-Atomen und Ester ein- oder mehrwertiger Alkohole mit Mono- oder Dicarbonsäuren. Geeignete Kohlenwasserstofföle sind z. B. Paraffinölel, Vaseline, Squalan und synthetische Kohlenwassserstoffe wie z. B. flüssige Polyolefine oder 1,3-Di-(2-ethylhexyl)-cyclohexan. Geeignete Silikonöle sind z. B. die linearen Polydimethylsiloxane und die cyclischen Silikone wie z. B. Decamethylcyclopentasiloxan oder Octamethylcyclotetrasiloxan. Geeignete flüssige Alkanole sind z. B. Oleyl- oder Erucylalkohol oder Guerbetalkohole wie z. B. 2-Hexyl-decanol oder 2-Octyldodecanol.

Geeignete Ester sind vor allem flüssige Verbindungen gemäß den Formeln I, II oder III

R¹-COOR² (I)

R²-OOC-R³-COOR² (II)

R¹-COO-R³-OOC-R¹ (III)

die mindestens 10 C-Atome enthalten und worin R¹ und R² Alkylgruppen mit 1-22 C-Atomen oder Alkenylgruppen mit 8 - 22 C-Atomen und R³ Alkylengruppen mit 2-16 C-Atomen sind. Ölkomponenten vom Typ der Mono- und Diester der Formeln I, II und III sind als kosmetische und pharmazeutische Ölkomponenten sowie als Gleit- und Schmiermittelkomponenten bekannt. Unter den Mono- und Diestern dieser Art kommt bei Raumtemperatur (20 °C) flüssigen Produkten die größte Bedeutung zu. Als Ölkomponenten geeignete Monoester (I) sind z. B. die Methylester und Isopropylester von Fettsäuren mit 12 - 22 C-Atomen, wie z. B. Methyllaurat, Methylstearat, Methyloleat, Methylerucat, Isopropylpalmitat, Isopropylmyristat, Isopropylpalmitat, Isopropylstearat, Isopropyloleat. Andere geeignete Monoester sind z. B. n-Butylstearat, n-Hexyllaurat, n-Decyloleat, Isoocrylstearat, Isononylpalmitat, Isononylisononanoat, 2-Ethylhexyl-palmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholgemischen und technischen aliphatischen Carbonsäuren erhältlich sind, z. B. Ester aus gesättigten und ungesättigten Fettalkoholen mit 12-22 C-Atomen und gesättigten und ungesättigten Fettsäuren mit 12 - 22 C-Atomen, wie sie aus tierischen und pflanzlichen Fetten zugänglich sind. Geeignet sind auch natürlich vorkommende Monoester- bzw. Wachsester-Gemische, wie sie z. B. im Jojobaöl oder im Spermöl vorliegen.

Geeignete Dicarbonsäuren (II) sind z. B. Di-n-butyl-adipat, Di-n-butyl-sebacat, Di-(2-ethylhexyl)-adipat. Di-(2-hexyldecyl)succinat und Di-isotridecyl-acelaat. Geeignete Diolester (III) sind z. B. Ethylenglycol-dioleat, Ethylenglycol-di-isotridecanoat, Propylenglycol-di-(2-ethyl-hexanoat), Butandiol-di-isostearat und Neopentylglycol-di-caprylat.

Auch flüssige Triglyceride eignen sich z. B. Glycerin-tricaprylat oder Triolein, ungesättigte natürliche Pflanzenöle wie z. B. Olivenöl, Sojaöl, Sonnenblumenöl, Erdnußöl, Rapsöl, Palmöl, Baumwollsaatöl, Distelöl, Mandelöl oder Sesamöl.

In einer besonders bevorzugten Auführung der Erfindung ist als nicht-essentielle, nicht-etherische Ölkomponente ein ungesättigter Wachsester mit 24-48 C-Atomen enthalten. Solche ungesättigten Wachsester können durch Veresterung von Fettsäuren mit 12-24 C-Atomen mit Fettalkoholen mit 8-24 C-Atomen erhalten werden. Dabei sollte wenigstens ein ungesättigter Fettalkohol oder eine ungesättigte Fettsäure zur Veresterung eingesetzt werden. Besonders bevorzugt geeignet ist ein natürlich vorkommender flüssiger Wachsester, das Jojobaöl. Dieses Öl setzt sich im wesentlichen aus Estern der Ölsäure, Jojobasäure (cis-11-Eicosensäure) und Erucasäure mit cis-11-Eicosenol, Erucaalkohol und cis-15-Tetracosenol zusammen. Es wird aus den bohnenartigen Samen des Jojobastrauchs (Simmondsia chinensis) gewonnen, der in Mexiko, Kalifornien und Arizona angepflanzt wird.

Weitere ebenfalls geeignete flüssige Ölkomponenten sind z. B. Dialkylether, z. B. Methyl-Stearylether, Butyl-stearylether oder Di-n-octylether, Glycerinmonoalkylether wie z. B. Glycerin-mono-(2-ethylhexyl)-ether und Di-n-alkylcarbonate wie z. B. Di-n-octylcarbonat und Di-n-laurylcarbonat.

Neben den flüssigen Ölkomponenten können auch feste oder wachsartige Fettstoffe, z. B. gehärtete Triglyceridwachse, feste Paraffine und feste Wachsester wie z. B. Bienenwachs, Apfelwachs, Sonnenblumenschalenwachs, Schellack oder gehärtetes Rizinusöl enthalten sein. Die Menge solcher Wachse sollte jedoch nur so hoch sein, daß das Gemisch aus Öl-und Wachskomponenten bei 20 °C flüssig bleibt.

Als Wirkstoffe enthalten die erfindungsgemäßen Zahnreinigungscremes therapeutisch wirksame Stoffe zur Bekämpfung von Karies, Zahnstein, Parodontitis oder anderen Zahn-und Zahnfleischerkrankungen. Ein bevorzugt enthaltener Wirkstoff ist eine karieshemmende Fluorverbindung, bevorzugt aus der Gruppe der Fluoride oder Monofluorphosphate in einer Menge von 0,1-0,5 Gew.-% Fluor. Geeignete Fluorverbindungen sind z. B. Natriumfluorid, Kaliumfluorid, Zinnfluorid, Natriummonofluorophosphat (Na₂ PO₃F), Kaliummonofluorphosphat oder das Fluorid einer organischen Aminoverbindung.

Bevorzugt geeignete therapeutische Wirkstoffe für die erfindungsgemäße Zahnreinigungscreme sind Antizahnstein-Wirkstoffe vom Typ der Organophosphonate. In einer besonders bevorzugten Ausführung ist daher als Wirkstoff ein Hydroxy- oder Aminoalkan-diphosphonsäure-Salz gegen Zahnsteinbildung und eine Fluorverbindung gegen Karies enthalten.

Geeignete Organophosphonate sind vor allem das 1-Hydroxyethan-1,1-diphosphonsäure-Na-Salz und das 1 -Azacydoheptan-2,2-diphosphonsäure-Na-Salz.

Weitere geeignete Wirkstoffe sind z. B.
- gegen Zahnstein wirksame kondensierte Phosphate wie z. B. Na-Tripolyphosphat, Na-Pyrophosphat oder Na-Trimetaphosphat
- antimikrobielle Plaque-Inhibitoren wie z. B. Hexachlorophen, Chlorhexidin, Hexetidin, Triclosan, Bromchlorophen, Phenylsalicylsäureester u. a.
- entzündungshemmende und wundheilende Wirkstoffe wie z. B. Allantoin, Harnstoff, Panthenol, Retinol, Azulen, Kamillewirkstoffe, Tranexamsäure, Acetylsalicylsäurederivate oder Rhodanid
- die Remineralisierung und den Verschluß von Dentalkanälchen fördernde Stoffe wie z. B. Dicalciumphosphat-dihydrat, bevorzugt in Kombination mit Magnesiumionen. Die Remineralisierung fördernde zweiwertige Metallsalze, insbesondere Salze des Magnesiums und des Zinks, z. B. Sulfate, Fluoride, Citrate oder Acetate dieser Metalle. Bevorzugt sind gelöste Salze des Magnesiums oder des Zinks oder einer Mischung beider Salze in einer Menge von 0,02 bis 0,2 Gew.-% Mg⁺⁺ und/oder Zn⁺⁺ enthalten.
- die Demineralisierung hemmende Komponenten wie z. B. Phosvitin oder andere Glycophosphoproteine.

Die genannten therapeutischen Wirkstoffe sind in den erfindungsgemäßen Zahnreinigungscremes in Mengen von 0,01 - 5 Gew.-% enthalten; dabei richtet sich die Menge nach der für eine ausreichend Wirkung erforderlichen Konzentration. Die bevorzugt enthaltenen Hydroxy-oder Aminoalkandiphosphonsäure-Salze sind bevorzugt in einer Menge von 0,1 -1 Gew.-% enthalten.

Die erfindungsgemäßen Zahnreinigungscremes enthalten Polierkomponenten, bevorzugt in einer Menge von 10- 30 Gew.-% und Feuchthaltemittel, bevorzugt in einer Menge von 15 - 40 Gew.-%. Die Art und Menge der eingesetzten Polierkomponenten hat dabei einen sehr entscheidenden Einfluß auf die Viskosität der Zahnreinigungscreme.

Als Polierkomponenten eignen sich alle für Zahnpasten bekannten Reibkörper wie z. B. Kieselsäure, Aluminiumhydroxid, Aluminiumoxid, Calciumpyrophosphat, Kreide, Natriumaluminiumsilikate (z. B. Zeolith A), Dicalciumphosphat-Dihydrat, organische Polymere, z. B. Polymethacrylat oder Gemische dieser Reibkörper.

Als Kieselsäurepoliermittel eignen sich alle als Putzkörper bekannten Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolg die Trocknung unter schonenden Bedingungen auf Wassergehalte von 15 - 35 Gew.-%, so werden die sogenannten Hydrogelkieselsäuren erhalten, wie sie z. B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20 °C) in einer Menge von 10 - 20 Gew.-% der Zahncreme. Bevorzugt geeignete Fällungskieselsäuren dieser Art weisen außerdem gerundete Ecken und Kanten auf und sind unter der Handelsbezeichnung Sident® 12 DS (DEGUSSA) erhältlich.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 - 14 µm bei einer spezifischen Oberfläche von 40 - 75 m²/g (nach BET) aus und eignen sich besonders gut für Zahnreinigungscremes gemäß der vorliegenden Erfindung.

Als weitere Poliermittelkomponente kann auch z. B Aluminiumoxid in Form von schwach kalzinierter Tonerde mit einem Gehalt an γ- und α-Aluminiumoxid in einer Menge von ca. 1 - 5 Gew.-% enthalten sein. Ein solches geeignetes Aluminiumoxid ist unter der Handelsbezeichnung "Poliertonerde P 10 feinst" (Giulini Chemie) erhältlich. Als besonders geeignet hat sich der Zusatz eines Poliermittels erwiesen, das selbst eine restaurierende Wirkung auf Läsionen und offene Dentalkanälchen hat, das Dicalciumphosphat-dihydrat. Dicalciumphosphat-dihydrat (CaHPO₄ •2H₂O) kommt in der Natur als Brushit vor und ist im Handel in geeigneten Korngrößen von 1 bis 50 µm erhältlich. Bevorzugt enthalten die erfindungsgemäßen Zahnreinigungscremes als Polierkomponente Kieselsäuren oder ein Gemisch aus Kieselsäuren und Aluminiumoxid im Gewichtsverhältnis 1 : (0,1-1) in einer Menge von 12 - 20 Gew.-%.

Als Bindemittel und Konsistenzregler können z. B. natürliche und / oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan-Gum, Succinoglycan-Gum, Johannisbrotmehl, Pectine, wasserlösliche Carboxyvinylpolymere (z.B. Carbopol® -Typen), Polyvinylalkohol, Polyvinylpyrrolidon, Polyethylenglycole, insbesondere solche mit Molekulargewichten von 1 500 - 1 000 000, eingesetzt werden.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren.

In einer bevorzugten Ausführung der Erfindung ist als Bindemittel ein mikrobielles Heteropolysaccharid, z. B. Xanthan-Gum oder ein Succinoglycan-Gum, in einer Menge von 0,5 - 2 Gew.-% in der Zahnreinigungscreme enthalten.

Eine weitere Verbesserung der Reinigungswirkung der erfindungsgemäßen Zahnreinigungsmittel kann man durch Zusatz eines geeigneten Tensids erzielen. Der Tensidzusatz kann auch zur Erzeugung eines Schaums beim Zähnebürsten, zur Stabilisierung der Poliermitteldispersion sowie zur Emulgierung oder Solubilisierung der Ölkomponenten erwünscht sein. Geeignete Tenside, die eine gewisse Schaumwirkung entfalten, sind die anionischen Tenside, z. B. Natriumalkylsulfate mit 12 - 18 C-Atomen in der Alkylgruppe. Diese Tenside haben auch eine gewisse enzymhemmende Wirkung auf den bakteriellen Stoffwechsel des Zahnbelags. Weitere geeignete Tenside sind Alkalisalze, bevorzugt Natriumsalze, von Alkylpolyglycolethersulfaten mit 12-16 C-Atomen in der linearen Alkylgruppe und 2-6 Glycolethergruppen im Molekül, von linearem Alkan-(C₁₂-C₁₈)-sulfonat, von Sulfobernsteinsäuremonoalkyl-(C₁₂-C₁₈)-estern, von sulfatierten Fettsäuremonoglyceriden, sulfatierten Fettsäurealkanolamiden, Sulfoessigsäurealkyl-(C₁₂C₁₆)-estem, Acylsarcosinen, Acyltauriden und Acylisethionaten mit jeweils 8 - 18 C-Atomen in der Acylgruppe.

Auch zwitterionische und ampholytische Tenside können, bevorzugt in Kombination mit anionischen Tensiden, eingesetzt werden. Geeignete zwitterionische Tenside sind z. B. Betaintenside wie z. B. das Kokosalkylbetain oder das Kokosacylamidopropylbetain. In einer bevorzugten Ausführung der Erfindung sind als oberflächenaktive Stoffe wenigstens ein C₁₀₋C₁₈-Alkylsulfat-Salz und ein Betaintensid enthalten. Zur Förderung der Reinigungswirkung eignet sich auch der Einsatz nicht-ionogener Tenside. Geeignete nicht-ionische Tenside sind z. B. die Anlagerungsprodukte von Ethylenoxid an Fettalkohole, an Fettsäuren, an Fettsäuremonoglyceride, an Sorbitan-Fettsäuremonoester oder an Methylglucosid-Fettsäuremonoester. Die angelagerte Menge an Ethylenoxid sollte dabei so hoch sein, daß die Tenside wasserlöslich sind, d. h. es sollte wenigstens 1 g/l in Wasser bei 20 °C löslich sein. Eine weitere Gruppe von geeigneten Tensiden sind die Alkyl-(oligo)-glcoside mit 8- 16 C-Atomen in der Alkylgruppe und einem Oligomerisationsgrad des Glycosidrestes von 1-4. Alkyl-(oligo)-glycoside, ihre Herstellung und Verwendung als oberflächenaktive Stoffe sind z. B. aus US-A-3,839,318, DE-A-20 36 472, EP-A-77 167 oder WO-A-93/10132 bekannt. Bezüglich des Glycosidrestes gilt, daß sowohl Monoglycoside (x=1), bei denen ein Monosaccaridrest glycosidisch an einem Fettalkohol mit 10 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl-(oligo)-glycosid ein Alkyl-(oligo)-glucosid der Formel RO(C₆H₁₀O)ₓ-H, in der R eine Alkylgruppe mit 12 bis 14 C-Atomen ist und x einen Mittelwert von 1 bis 4 hat.

Zur Solubilisierung der wasserunlöslichen Aromaöle kann ein nicht-ionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäure-sorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzeöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton.

Als weitere Hilfsmittel können in den erfindungsgemäßen Zahnreinigungscremes auch Süßungsmittel enthalten sein. Geeignete Süßungsmittel sind z. B. Saccharin-Natrium, Acesulfam-K, Aspartame® , Natrium-Cyclamat, Stevioside, Thaumatin, Sucrose, Lactose, Maltose, Fructose und Glycyrrhizin. Schließlich können weitere übliche Hilfsmittel zur Verbesserung der Stabilität und der sensorischen Eigenschaften der Zahnreinigungscremes enthalten sein. Solche Hilfsmittel sind z. B.
- Konservierungsstoffe, z. B. p-Hydroxybenzoesäureester, Natriumsorbat, Natriumbenzoat
- Puffersalze, z. B. primäre, sekundäre und/oder tertiäre Alkaliphosphate oder Citronensäure/Natriumcitrat,
- Farbstoffe und Pigmente, z. B. TiO₂ oder Glimmer oder pigmentierte Kieselsäuregranulate,
- niedere Alkohole wie z. B. Ethanol oder Isopropanol.

In einer bevorzugten Ausführung enthalten die erfindungsgemäßen Zahnreinigungscremes als weitere Hilfsmittel ein Di- und/oder Trialkaliorthophosphat-Salz in einer Menge von 1 - 5 Gew.-%. Der Wassergehalt der erfindungsgemäßen Zahnreinigungscremes liegt bei 35 - 60 Gew.-%, bevorzugt bei 40 - 50 Gew.-%. Die Herstellung der erfindungsgemäßen Zahnreinigungscremes erfolgt wie üblich durch Dispergieren der Polierkomponenten in einem Gemisch aus Wasser und Feuchthaltemitteln und aufeinanderfolgendes Einmischen einer Dispersion des Bindemittels in Wasser und/oder Propylenglycol, der pulverförmigen Zusätze und Hilfsmittel, der Tenside, Ölkomponenten und der Aromaöle. Die Viskosität läßt sich dabei durch die Menge der Poliermittelkomponente, der Bindemittel und der Viskositätsregulatoren in weiten Bereichen steuern. Sie wird erfindungsgemäß auf einem Wert im Bereich von 50 - 200 Pa·s eingestellt. Dabei wählt man bevorzugt eine Viskosität im unterem Bereich, da bei Lagerung die Viskosität leicht ansteigen kann.

Die Messung der Viskosität erfolgt mit einem Rotationsviskosimeter bevorzugt des Typs Brookfield RVF Helipath unter Verwendung der Spindel TE bei 4 UpM (Umdrehungen pro Minute). Die Endviskosität (nach ca. 24 Stunden) bei 20 °C liegt bevorzugt in einem Bereich von 60 - 125 Pa·s.

Die erfindungsgemäße Zahnreinigungscreme eignet sich aufgrund dieser Viskosität besonders gut für die Dosierung aus einer flexiblen Spendetube mit Schlitzventil, die in "Überkopf"-Position gelagert wird. Durch den Gehalt an dispergierten, nicht-essentiellen und nicht-etherischen Ölkomponenten weist sie eine gute Duktilität und Gleitfähigkeit auf und neigt nicht zur Bildung harter Krusten im Ventilbereich.

Ein weiterer, bevorzugter Gegenstand der Erfindung ist daher ein Zahnreinigungsprodukt in Form einer flexiblen Spendetube mit Schlitzventil und einer als Standauflage zur ÜberkopfLagerung dienenden Ventilabdeckung, die mit einer erfindungsgemäßen Zahnreinigungscreme gefüllt ist. Spendetuben dieser Art sind z. B. Gegenstand der deutschen Patentanmeldung DE 100 23 244.2.

Die folgenden Beispiele sollen den Gegenstand der Erfindung näher erläutern:

### Beispiele:

Es wurden Zahnreinigungscremes der in den Beispielen 1- 4 (Tabelle I) angegebenen Zusammensetzung herstellt.

Dazu wurden Sorbit, Glycerin und ca. 80 % des Wassers miteinander gemischt, die Lösung von AHP 100 und Natriumhydroxid in ca. 10 % des Wassers zugemischt, die Polierkomponenten in dem Ansatz dispergiert, Saccharin, Fluorid, die Orthophosphate zugemischt, eine Lösung der oberflächenaktiven Stoffe in ca. 10 % des Wassers und eine Dispersion des Heteropolysaccharids in 1,2-Propylenglycol zugemischt. Schließlich wurde das Aroma und das Jojobaöl untergemischt und 30 Minuten gerührt. Nach 4 Stunden Lagerung bei 25 °C wurden die Zahnreinigungscremes in eine Standtube mit Schlitzventil eingefüllt. Beim Ausdrücken der Zahncremes aus der Standtube wurde ein stabiler, weicher Zahncremestrang von guter Formbeständigkeit und hohem Glanz erhalten.

**Tabelle I**

| | **1** | **2** | **3** | **4** |
|---|---|---|---|---|
| Sident® 8 | 14,0 | 14,0 | 14,0 | 14,0 |
| Sorbosil® AC 33 | 1,0 | 1,0 | - | - |
| Poliertonerde P10 feinst | - | - | 1,0 | 1,0 |
| Glycerin (86%ig DAB) | 18,0 | 18,0 | 18,0 | 18,0 |
| Sorbit (70%ig) | 14,0 | 14,0 | 14,0 | 14,0 |
| 1,2-Propylenglycol | 5,0 | 5,0 | 5,0 | 5,0 |
| AHP 100 | 0,5 | 0,5 | 0,5 | 0,5 |
| Na F | 0,32 | 0,32 | 0,32 | 0,32 |
| Saccharin-Na | 0,2 | 0,2 | 0,2 | 0,2 |
| Na₂HPO₄ | 1,5 | 1,5 | 1,5 | 1,5 |
| Na₃PO₄ | 0,5 | 0,5 | 0,5 | 0,5 |
| Ti O₂ | - | - | 0,5 | 0,5 |
| Timiron® Diamond Cluster MP 149 | 0,2 0,2 | 0,2 0,2 | - | - |
| Brillant Blue 1 %ig (Acid Blue 9) | 0,22 | 0,22 | - | - |
| pHB-Methylester | 0,1 | 0,1 | 0,1 | 0,1 |
| Keltrol® F | 1,25 | 1,25 | 1,25 | 1,25 |
| Texapon® K1296 | 1,5 | 1,5 | 1,5 | 1,5 |
| Tego Betain® BL 215 | 0,6 | 0,6 | 0,6 | 0,6 |
| Triclosan | 0,3 | - | 0,3 | - |
| Jojobaöl | 1,0 | 0,5 | 1,0 | 0,5 |
| Aromaöl | 1,15 | 1,15 | 1,0 | 1,0 |
| NaOH | 0,158 | 0,158 | 0,158 | 0,158 |

| Wasser | ad 100 | ad 100 | ad 100 | ad 100 |
|---|---|---|---|---|
| Viskosität (20 °C, Brookfield, Type RVF Helipath, Spindel TE, 4 UpM; nach 1 Std.) | 80 Pa·s | 70 Pa·s | 75 Pa·s | 75 Pa·s |

### Es wurden die folgenden Handelsprodukte eingesetzt

- Sident® 8 (Degussa) Kieselsäure, BET: 60 m²/g Mittlere Teilchenfeinheit 8-12 µm Schüttdichte: 300 g/l
- Sorbosil® AC 33 (Crosfield) Kieselsäure, BET: 300 m²/g Mittlere Teilchenfeinheit 7 µm Schüttdichte: 38 g/l
- Poliertonerde P10 feinst y-Aluminiumoxid (Giulini)
- AHP 100 Azacycloheptan-2,2-diphosphonsäure (Albright Wilson)
- Timiron® Diamond Glimmer und TiO₂ (8:1) Cluster MP 149 (Merck) Teilchenfeinheit: 20-150 µm Schüttdichte: 350 g/l
- Keltrol® F Xanthan Gum
- Texaponlr K1296 Na-Laurylsulfat (Cognis Deutschland)
- Tego Betain® BL 215 1-Kokosacylamino-3-dimethylaminopropan-3-(Goldschmidt) carboxymethylbetain

## Patentansprüche

1. Zahnreinigungscreme in Form einer wässrigen, fließfähigen Dispersion mit einem Gehalt an Poliermitteln, Feuchthaltemitteln, Bindemitteln, oberflächenaktiven Stoffen, Wirkstoffen, Aromen und weiteren Hilfsmitteln, **dadurch gekennzeichnet, dass** 0,1-2 Gew.-% wenigstens einer bei 20 °C flüssigen, nichtessentiellen und nichtetherischen Ölkomponente darin dispergiert ist und dass die Viskosität bei 20 °C im Bereich von 50 - 200 Pa·s liegt, wobei als Feuchthaltemittel Glycerin, Sorbit und 1,2-Propylenglycol in einem Gewichtsverhältnis von 1 : (0,5-1) : (0,1-0,5) und in einer Gesamtmenge von 20 - 40 Gew.-% enthalten sind.

2. Zahnreinigungscreme gemäß Anspruch 1, **dadurch gekennzeichnet, daß** als Wirkstoffe ein Hydroxy- oder Aminoalkan-diphosphonsäure-Salz gegen Zahnsteinbildung und eine Fluorverbindung gegen Karies enthalten sind.

3. Zahnreinigungscreme gemäß einem der Ansprüche 1 oder 2, **dadurch gekennzeichnet, dass** als nichtessentielle, nichtetherische Ölkomponente ein ungesättigter Wachsester mit 24 - 48 C-Atomen, bevorzugt Jojobaöl, enthalten ist.

4. Zahnreinigungscreme gemäß einem der Ansprüche 1-3, **dadurch gekennzeichnet, dass** als Polierkomponenten Kieselsäuren oder ein Gemisch aus Kieselsäuren und Aluminiumoxid im Gewichtsverhältnis 1 : (0,1-1) in einer Menge von 12 - 20 Gew.-% enthalten sind.

5. Zahnreinigungscreme gemäß einem der Ansprüche 1-4 **dadurch gekennzeichnet, dass** als oberflächenaktive Stoffe wenigstens ein C₁₀-C₁₈-Alkylsulfat-Salz und ein Betaintensid enthalten sind.

6. Zahnreinigungscreme gemäß einem der Ansprüche 1 - 5, **dadurch gekennzeichnet, daß** als Bindemittel ein mikrobielles Heteropolysaccharid enthalten ist.

7. Zahnreinigungscreme gemäß einem der Ansprüche 1 - 6, **dadurch gekennzeichnet, daß** als weiteres Hilfsmittel ein Di- und / oder Trialkali-orthophosphat in einer Menge von 1 - 5 Gew.-% enthalten ist.

8. Zahnreinigungsprodukt in Form einer flexiblen Spendetube mit Schlitzventil und einer als Standauflage zur "Überkopf"-Lagerung dienenden Ventilabdeckung, **dadurch gekennzeichnet, daß** die Spendetube mit einer Zahnreinigungscreme gemäß einem der Ansprüche 1 - 8 gefüllt ist.

## Claims

1. Dental cleaning cream in the form of an aqueous, flowable dispersion with a content of polishes, humectants, binders, surface-active substances, active ingredients, aromas and further auxiliaries, **characterized in that** 0.1-2% by weight of at least one non-essential and non-ethereal oil component liquid at 20°C is dispersed therein and that the viscosity at 20°C is in the range 50-200 Pa·s, where glycerol, sorbitol and 1,2-propylene glycol are present as humectant in a weight ratio of 1: (0.5-1) : (0.1-0.5) and in a total amount of 20-40% by weight.

2. Dental cleaning cream according to Claim 1, **characterized in that** a hydroxy- or aminoalkanediphosphonic acid salt to combat the formation of tartar, and a fluorine compound to combat caries are present as active ingredients.

3. Dental cleaning cream according to one of Claims 1 or 2, **characterized in that** an unsaturated wax ester with 24-48 carbon atoms, preferably jojoba oil, is present as non-essential, non-ethereal oil component.

4. Dental cleaning cream according to one of Claims 1-3, **characterized in that** silicas or a mixture of silicas and aluminium oxide are present as polishing components in the weight ratio 1:(0.1-1) in an amount of 12-20% by weight.

5. Dental cleaning cream according to one of Claims 1-4, **characterized in that** at least one C₁₀-C₁₈₋alkyl sulphate salt and a betaine surfactant are present as surface-active substances.

6. Dental cleaning cream according to one of Claims 1-5, **characterized in that** a microbial heteropolysaccharide is present as binder.

7. Dental cleaning cream according to one of Claims 1-6, **characterized in that** a di- and/or trialkali metal orthophosphate is present as further auxiliary in an amount of 1-5% by weight.

8. Dental cleaning product in the form of a flexible dispensing tube with slit valve and a valve cover serving as standing rest for "overhead" storage, **characterized in that** the dispensing tube is filled with a dental cleaning cream according to one of Claims 1-8.

## Revendications

1. Crème dentifrice sous la forme d'une dispersion aqueuse apte à s'écouler contenant des agents de polissage, des agents de maintien de l'humidité, des liants, des substances tensioactives, des substances actives, des arômes et d'autres adjuvants, **caractérisée en ce que** 0,1 à 2 % en poids d'au moins un composant d'huile liquide à 20 °C, non essentielle et non éthérée y est dispersée, et **en ce que** la viscosité à 20 °C se situe dans la plage de 50 à 200 Pa.s, du glycérol, du sorbitol et du 1,2-propylène glycol y étant contenu à titre d'agent de maintien de l'humidité, dans un rapport pondéral de 1 : (0,5-1) : (0,1-0,5) et en une quantité totale de 20 à 40 % en poids.

2. Crème dentifrice selon la revendication 1, **caractérisée en ce qu'**elle contient, à titre de substances actives, un sel d'acide hydroxy- ou amino-alcane-diphosphonique contre le dépôt de tartre sur les dents et un composé fluoré contre les caries.

3. Crème dentifrice selon l'une quelconque des revendications 1 ou 2, **caractérisée en ce qu'**elle contient, à titre de composant d'huile non essentielle, non éthérée, un ester de cire insaturé contenant de 24 à 48 atomes de carbone, de préférence de l'huile de jojoba.

4. Crème dentifrice selon l'une quelconque des revendications 1 à 3, **caractérisée en ce qu'**elle contient, à titre de composant de polissage, des acides siliciques ou un mélange d'acides siliciques et d'oxyde d'aluminium dans un rapport pondéral de 1: (0,1-1) en une quantité de 12 à 20 % en poids.

5. Crème dentifrice selon l'une quelconque des revendications 1 à 4, **caractérisée en ce qu'**elle contient, à titre de substances tensioactives, au moins un sel de sulfate d'alkyle en C₁₀-C₁₈ et un agent tensioactif à base de bétaïne.

6. Crème dentifrice selon l'une quelconque des revendications 1 à 5, **caractérisée en ce qu'**elle contient, à titre de liant, un hétéropolysaccharide microbien.

7. Crème dentifrice selon l'une quelconque des revendications 1 à 6, **caractérisée en ce qu'**elle contient, à titre d'adjuvant supplémentaire, un orthophosphate dialcalin et/ou trialcalin en une quantité de 1 à 5 % en poids.

8. Produit de dentifrice sous la forme d'un tube de distribution flexible comportant une valve à fente et un recouvrement de valve faisant office de support d'appui pour l'entreposage « à l'envers », **caractérisé en ce que** le tube de distribution est rempli avec une crème dentifrice selon l'une quelconque des revendications 1 à 7.
